(19) Europäisches Patentamt / European Patent Office / Office européen des brevets

(11) **EP 3 277 251 B1**

(12) **FASCICULE DE BREVET EUROPEEN**

(45) Date de publication et mention de la délivrance du brevet:
**29.05.2019 Bulletin 2019/22**

(21) Numéro de dépôt: **16709358.2**

(22) Date de dépôt: **08.03.2016**

(51) Int Cl.:
*A61K 8/34* (2006.01)     *A61K 8/60* (2006.01)
*A61Q 11/00* (2006.01)     *A61K 8/96* (2006.01)
*A61K 8/98* (2006.01)     *A61K 9/10* (2006.01)

(86) Numéro de dépôt international:
**PCT/EP2016/054935**

(87) Numéro de publication internationale:
**WO 2016/155992 (06.10.2016 Gazette 2016/40)**

(54) **SOLUTION IONIQUE AQUEUSE, À BASE DE SELS MINÉRAUX DISSOUS, NOTAMMENT DESTINÉE AUX SOINS DE LA GORGE**

WÄSSRIGE IONISCHE LÖSUNG AUS GELÖSTEN MINERALSALZEN, INSBESONDERE ZUR RACHENPFLEGE

AQUEOUS IONIC SOLUTION, MADE FROM DISSOLVED MINERAL SALTS, IN PARTICULAR INTENDED FOR THROAT CARE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.04.2015 FR 1552862**

(43) Date de publication de la demande:
**07.02.2018 Bulletin 2018/06**

(73) Titulaire: **Laboratoire de la Mer**
**35400 Saint-Malo (FR)**

(72) Inventeurs:
• **BERTAUD, Olivier**
  **35400 Saint-Malo (FR)**
• **BEAULIEU, Anne**
  **35400 Saint-Malo (FR)**
• **MIMOT, Ludovic**
  **35400 Saint-Malo (FR)**

(74) Mandataire: **Cabinet Le Guen Maillet**
**3, impasse de la Vigie**
**CS 71840**
**35418 Saint-Malo Cedex (FR)**

(56) Documents cités:
**FR-A1- 2 971 713     US-A- 5 922 324**

EP 3 277 251 B1

**Description**

La présente invention concerne une solution ionique aqueuse, à base de sels minéraux dissous, notamment destinée aux soins de la gorge, en particulier au traitement, à la prévention et au soulagement des manifestations liées aux maux de gorge.

Le mal de gorge est un symptôme aux multiples origines, infectieuses ou non infectieuses. Les maux de gorge d'origine non infectieuse sont liés au contact des muqueuses ou de la sphère ORL avec des substances irritantes telles que le tabac, l'alcool, la pollution ou avec des allergènes. Les maux de gorge d'origine infectieuse résultent de l'exposition des muqueuses de la sphère ORL avec des virus ou des bactéries.

La pharyngite est une des pathologies d'origine infectieuse associées aux maux de gorge. C'est une inflammation du pharynx. C'est l'affection ORL la plus commune qui touche l'ensemble de la population, des plus petits aux plus grands. Elle présente des signes locaux peu différents de l'angine, notamment, des maux de gorge, de la fièvre et des difficultés à avaler. La pharyngite est souvent associée à un rhume. Elle peut aussi s'accompagner d'une otite aiguë, d'une sinusite aiguë et/ou d'une bronchite aiguë. Elle est le plus souvent d'origine virale. Chez l'enfant, elle se développe essentiellement dans la partie supérieure du pharynx, le rhino-pharynx. La rhinopharyngite aiguë est l'affection la plus courante chez l'enfant.

L'angine est une inflammation d'origine infectieuse des amygdales, voire de l'ensemble de l'oropharynx, qui deviennent rouges et enflées, parfois couvertes de points blancs ou jaunâtres. Elle se manifeste au début par des picotements ressentis dans la gorge, une gêne plus ou moins importante à la déglutition, voire une déglutition douloureuse, une sensation de brûlures dans la gorge, une tendance à toussoter et à se racler la gorge lors de la formation de mucus, parfois de la toux et une douleur irradiant jusqu'aux oreilles. Dans 80 % des cas, l'angine est d'origine virale et bénigne. C'est une maladie fréquente et banale. Seules 20 % des angines sont d'origine bactérienne et doivent bénéficier d'un traitement antibiotique. L'angine bactérienne présente un pic de fréquence chez l'enfant entre 5 et 15 ans. Elle est plus rare chez l'enfant de moins de 3 ans ainsi que chez l'adulte.

Les principaux agents infectieux impliqués dans les infections de la sphère ORL, telles que la pharyngite et l'angine, sont des virus pour 40 % des cas et des bactéries pour 30 % des cas. Concernant les virus, il s'agit majoritairement de rhinovirus, d'adénovirus, de virus respiratoire syncytial, ou de l'Influenza A et B. Il peut s'agir également du virus de l'herpès avec une incidence plus faible, mais des épisodes plus sévères en termes de symptômes.

Concernant les bactéries, il s'agit majoritairement de bactéries à streptocoques β-hémolytiques du groupe A (GABHS, aussi appelé S. Pyogenes) et plus rarement de S. Viridans, M. Pneumoniae, C. Pneumoniae, H. Influenzae.

Il faut noter que dans 30 % des cas, l'origine infectieuse n'est pas connue ou identifiée.

Non-traitées, les angines et les pharyngites peuvent être source de complications locales péri-pharyngées : surinfection bactérienne, amygdalite, phlegmon périamygdalien par exemple.

Le traitement de ces pathologies, telles que la pharyngite et l'angine d'origine virale, est symptomatique. Seules les infections streptococciques sont traitées avec des antibiotiques. Un prélèvement suivi d'un test rapide est réalisé chez le médecin généraliste pour déterminer la nature du traitement.

En ce qui concerne les principes actifs utilisés à ce jour, la plupart des préparations médicamenteuses les plus utilisées contiennent :

- un antiseptique seul, tel que l'hexamidine, l'acétarsol sodique, la chlorhexidine, l'hexitidine, le chlorure de benzalkonium, etc...
- un antiseptique associé à un anesthésique, tel que la tétracaïne, la lidocaïne, le butoforme, etc...
- un anti-inflammatoire, tel que le lysozyme, l'alpha-amylase, la papaïne, la ribonucléase, etc....

Les traitements des maux de gorge à partir de tels composés présentent de multiples inconvénients :
Les antiseptiques locaux sont sans effet sur la douleur pharyngée, ne présentent pas d'action adoucissante, sont soumis à une posologie et durée d'utilisation limitées. Une utilisation répétée d'antiseptique peut engendrer un déséquilibre de la flore commensale de la cavité bucco-pharyngée qui assure un rôle primordial dans le contrôle des infections et le système immunitaire. Les antiseptiques ne ciblent pas un seul pathogène mais fragilisent l'ensemble de la flore résidente. Les antiseptiques locaux rallongent la durée de reformation d'un biofilm « sain » qui participerait à la protection de la muqueuse exposant les sujets convalescents à un risque de récidive.

Les anesthésiques locaux associés à des antiseptiques locaux peuvent s'avérer dangereux au niveau de la gorge où ils sont susceptibles d'entraîner des « fausses routes » lors de la déglutition liée à l'alimentation, par anesthésie du carrefour oro-pharyngé. Ils doivent être utilisés à distance des repas et des boissons. Ils sont notamment déconseillés chez l'enfant de moins de 12 ans et la personne âgée. La posologie et la durée d'utilisation sont limitées. En outre, ils empêchent le bon fonctionnement du tapis muco-ciliaire en bloquant le mouvement des cils. Ils laissent stagner le mucus infecté à la surface de la muqueuse amplifiant le phénomène inflammatoire. Ils déséquilibrent la flore microbienne normale de la cavité buccale avec un risque de diffusion bactérienne ou fongique. Un traitement répété ou prolongé au niveau de la muqueuse peut exposer aux risques d'effets systémiques toxiques des anesthésiques de contact (atteinte du système nerveux central avec convulsions, dépression du système cardiovasculaire). L'attention des sportifs sera attirée sur le fait que cette spécialité contient un principe actif, l'anesthésique local, pouvant induire une réaction positive des tests pratiqués lors des contrôles antidopage.

Les anti-inflammatoires ne présentent pas d'action antiseptique, sont sans effet sur la douleur et n'ont aucun intérêt en cas d'infection d'origine virale. Leur action se produit sur l'oedème pharyngé. Là encore, la posologie et la durée d'utilisation sont limitées.

Concernant des produits d'origine naturelle, certains produits proposés sont élaborés à partir de composés issus de plantes ou de produits de la ruche: extraits de thé vert, camomille, pin sylvestre, miel, huiles essentielles, concentré de citron. Les produits phytothérapeutiques proposés aujourd'hui ont une action essentiellement adoucissante, en manque d'efficacité instantanée et/ou sur la douleur sur le long terme.

Concernant la galénique, la plupart des préparations médicamenteuses ou phytothérapeutiques utilisées se présentent sous la forme de pastilles et de collutoires (spray). Environ 60% de la population française déclarent utiliser des pastilles pour soulager les maux de gorge et environ 40% des sprays.

La galénique d'un tel produit pour la gorge est une caractéristique importante, en lien avec l'efficacité du produit. En effet, l'utilisation de pastilles chez les jeunes enfants et les personnes âgées n'est pas adaptée et déconseillée. Concernant les formulations liquides aptes à être délivrées en spray, les solutions doivent se maintenir sous une forme stable dans le temps, sans déphasage ni précipitation, et sans contamination microbienne. Une difficulté majeure provient du fait que certaines compositions liquides, par exemple en phase aqueuse, incluent des composés actifs lipophiles. Afin de permettre l'affinité et la solubilité des composants entre eux il est nécessaire d'ajouter des adjuvants, tels que des émulsifiants ou des tensioactifs. Certains produits contiennent ainsi de tels adjuvants, souvent d'origine non naturelle, à caractère allergisant ou irritant. De tels composés sont de plus susceptibles de modifier le goût du produit, le rendant inadapté à un traitement de longue durée. Leur sélection lors de la formulation d'un produit destiné à être ingéré par voie orale et mis en contact avec les muqueuses est particulièrement délicate.

Par ailleurs, les compositions formulées à partir de phases aqueuses sont propices à la contamination par des microorganismes. La contamination microbienne peut se produire lors de la fabrication ou du conditionnement (contamination primaire) ou lors de la conservation et de l'utilisation des compositions (contamination secondaire). Elles doivent donc contenir un ou plusieurs conservateurs. De tels conservateurs sont souvent dérivés de l'industrie de la chimie ce qui n'est pas souhaitable lorsque l'on envisage un produit naturel. De plus, certains conservateurs sont susceptibles d'avoir des effets indésirables lorsque leur concentration est trop importante, tels que des réactions allergiques ou d'autres formes d'intolérance. La mise au point de formulations exemptes de tels composés serait ainsi tout à fait intéressante.

Les trois principaux critères de choix chez les utilisateurs de produits pour les maux de gorge sont les suivants: l'action immédiate, l'efficacité pour traiter et soulager les maux de gorge et l'action prolongée sur une longue durée. S'ajoute à cela la volonté de disposer de solutions naturelles non toxiques à long terme. De plus, de tels produits doivent nécessairement être stables dans le temps, et d'utilisation facile, à tout moment de la journée, chez l'enfant, même très jeune, et l'adulte.

La société demanderesse s'est intéressée aux solutions ioniques salines dans le traitement des maux de gorge. Des solutions hypertoniques à base d'eau de mer, telles que celles décrites dans la demande de brevet européen EP 2 068 896 A1 appartenant à la société demanderesse, présentent des propriétés décongestionnantes des muqueuses intéressantes dans le cadre du soin de la gorge. En application sur la muqueuse de la gorge, elles permettent de réduire l'oedème pharyngé par action osmotique, de fluidifier l'excès de mucus, et d'évacuer les agents pathogènes et les médiateurs de l'inflammation par action mécanique. Ainsi, l'évacuation des pathogènes et du mucus est ici réalisée par fluidification et déglutition. Toutefois, il n'y a pas réellement d'activité antiseptique ou antivirale à proprement parler. De plus, elles ne présentent pas d'action efficace immédiate contre la douleur. En outre, de telles solutions hypertoniques présentent une viscosité proche de celle de l'eau pure et s'avèrent trop liquides pour adhérer efficacement aux muqueuses durant un temps d'action significatif. FR2971713A1 décrit une solution ionique aqueuse comprenant une solution hypertonique de sels minéraux présentant une osmolalité supérieure à 350 mOsm/kg, du sorbitol et du monopropyleneglycol.

La présente invention a pour objectif de pallier l'ensemble des inconvénients présentés en lien avec les produits précités.

En particulier, la présente invention a pour but de proposer une solution ionique aqueuse élaborée à partir de composés d'origine naturelle, qui soit efficace dans le traitement ou la prévention des douleurs de la gorge et de leurs manifestations, en particulier d'origine infectieuse, qui soit stable dans le temps sans induire de phénomènes de déphasage ou de précipitation malgré l'incorporation de composés non miscibles à une phase aqueuse, qui soit efficace à l'encontre des contaminations primaires ou secondaires, qui soit utilisable chez l'adulte comme chez le jeune enfant, sans présenter un goût défavorable à l'ingestion, sans modifier l'haleine de manière défavorable, qui soit apte à être délivrée en spray ou autre collutoire, qui se montre efficace contre la douleur de manière rapide, immédiatement comme sur le long terme, qui soit adhérente aux muqueuses sans générer de sensations d'étouffement ni de fausses routes ou quelconque gêne à la déglutition, qui puisse être utilisée plusieurs fois par jour, par exemple, au moins 6 fois par jour, et qui ne génère pas de conséquences négatives du fait de sa composition sur la santé générale.

Un autre but de la présente invention est de proposer une telle solution ionique aqueuse qui soit qualifiée de multi-action, c'est-à-dire qui traite à la fois plusieurs manifestations connues du mal de gorge, notamment, la douleur, l'inflammation, la présence de foyers bactériens ou viraux, la déglutition difficile, la sensation de brûlures et d'irritations et l'enrouement, la toux. En particulier, l'action anti-inflammatoire, antiseptique, l'action osmotique, l'action mucolytique et fluidifiante, l'action adoucissante, calmante et apaisante sera recherchée.

A cet effet, l'invention concerne une solution ionique aqueuse, notamment destinée aux soins de la gorge, comprenant :

- à titre de solvant aqueux, au moins 20 %, en poids par rapport au poids total de la composition, d'une solution hypertonique de sels minéraux présentant une osmolalité supérieure à 350 mOsm/kg,
- au moins 50 % d'un ou plusieurs polyols d'origine végétale, en poids par rapport au poids total de la composition,
- au moins un ester de saccharose et d'acide gras en C8-C24,
- un extrait de propolis et/ou de miel.

Avantageusement, la solution ionique aqueuse comprend entre 30 et 40%, plus préférentiellement entre 30 et 35%, en poids par rapport au poids total de la composition, de ladite solution hypertonique de sels minéraux présentant une osmolalité supérieure à 350 mOsm/kg.

Une solution hypertonique est une solution saline d'une osmolalité plus grande que celle du sang, donc supérieure à 300 mOsm/kg, environ. Une telle solution exerce une pression osmotique supérieure à celle du plasma sanguin normal. Par exemple, une solution de chlorure de sodium (NaCl) dont la concentration est supérieure à 9 g/L est une solution hypertonique.

La solution hypertonique de sels minéraux utilisée en tant que solvant aqueux dans le cadre de la présente invention est choisie dans le groupe constitué de l'eau de mer pure, de l'eau de mer diluée et des solutions aqueuses contenant des sels, en particulier du chlorure de sodium, et contenant en outre éventuellement au moins un autre sel choisi parmi ceux naturellement contenus dans l'eau de mer. A ce titre on citera, autre le chlorure de sodium, le chlorure de magnésium, le sulfate de magnésium, le sulfate de calcium, le sulfate de potassium, le carbonate de calcium, le bromure de magnésium. La composition de l'eau de mer est indiquée dans le livre Handbook of Chemistry and Physics 63ème édition, 1982-1983, page F 163, CRC PRESS.

Avantageusement, la solution hypertonique de sels minéraux est une solution contenant entre 22 et 25 g/L, préférentiellement entre 22 et 23 g/L, de sels minéraux dissous de sodium, de chlore, de sulfate, de magnésium, de calcium et de potassium.

Dans un mode de réalisation préféré de l'invention, la solution hypertonique de sels minéraux est à base d'eau de mer, et présente :

- une osmolalité supérieure à 350 mOsm/kg, préférentiellement comprise entre 500 et 2000 mOsm/kg, de préférence entre 550 et 700 mOsm/kg, et plus préférentiellement encore entre 600 et 650 mOsm/kg,
- une composition du point de vue ionique qui est qualitativement celle de l'eau de mer et les concentrations en $Na^+$, $K^+$, $Mg^{2+}$, $Ca^{2+}$ et $Cl^-$ suivantes :
- pour $Na^+$, de 5000 à 19000, de préférence de 5500 à 8000, et plus préférentiellement encore de 6000 à 6500 mg/1
- pour $K^+$, de 50 à 650, de préférence de 100 à 250 mg/1
- pour $Mg^{2+}$, de 1000 à 4000, de préférence de 1000 à 2000, et plus préférentiellement encore de 1100 à 1500 mg/1
- pour $Ca^{2+}$, de 300 à 1200, de préférence de 350 à 450 mg/1
- pour $Cl^-$, de 8000 à 33000, de préférence de 9000 à 15000, et plus préférentiellement encore de 10000 à 13000 mg/1.

Avantageusement, une telle solution hypertonique préférée contient également du brome (Br), de l'aluminium (Al), du fluor (F), de l'iode (I), du fer (Fe), du zinc (Zn), du cuivre (Cu), du manganèse (Mn), du sélénium (Se).

Une telle solution hypertonique à base d'eau de mer, que l'on nommera « solution hypertonique de référence » dans la suite de la présente description, peut être obtenue par dilution avec de l'eau distillée. Toutefois, de manière particulièrement avantageuse, une telle solution est obtenue par un procédé incluant une ou plusieurs étapes d'électrodialyse de manière à ajuster les concentrations des éléments ioniques. Une telle solution ainsi que son procédé de préparation sont déjà décrits dans la demande de brevet EP-A1-2 068 896.

La solution de sels minéraux hypertoniques agit au niveau de la gorge en particulier sur l'oedème, par effet osmotique, de manière immédiate. Elle agit également efficacement pour éliminer les bactéries ou les virus et les médiateurs de l'inflammation par action mécanique notamment. Elle fluidifie et lyse le mucus, facilite son élimination et favorise la clairance muco-ciliaire. Elle participe aux mécanismes de cicatrisation de la muqueuse.

La solution ionique comprend encore des polyols d'origine végétale en une quantité importante. A ce titre et de manière préférée, la solution comprend entre 50 et 65% dudit ou desdits polyols d'origine végétale, en poids par rapport au poids total de la composition.

On entend par « origine végétale », un composé issu d'une source végétale préférentiellement renouvelable.

A titre de polyols, on citera, le glycérol, le sorbitol, le maltitol, mannitol, le xylitol.

Selon un mode de réalisation préféré, les polyols d'origine végétale choisis sont le glycérol pour au moins 90 %, préférentiellement au moins 95 % et le sorbitol pour 5 à 10 %, en poids par rapport au poids total desdits polyols.

La solution ionique aqueuse selon l'invention comprend au moins 20 % d'eau de mer ce qui représente un milieu favorable à la contamination par des microorganismes, tels que des bactéries, lors de sa fabrication ou de son conditionnement (contamination primaire) ou lors de sa conservation et de son utilisation chez le consommateur. La quantité de polyols incluse dans la solution ionique permet de diminuer l'activité de l'eau et d'empêcher la prolifération de microorganisme tout en s'affranchissant de l'ajout de conservateurs.

Les polyols, en particulier le glycérol, agissent encore à titre d'humectant, apte à retenir l'humidité au niveau des muqueuses sur lesquelles la solution est destinée à être appliquée. Cette fonction participe à l'action anti douleur, à faciliter la déglutition, à diminuer les sensations de brûlures, de picotements et d'irritations.

Le propolis et/ou le miel présentent en particulier une action calmante et adoucissante au niveau des muqueuses. Le miel est avantageusement du miel d'acacia. L'extrait de propolis ne contient préférentiellement pas d'alcool, lequel est irritant pour les muqueuses.

Avantageusement, la solution comprend entre 0,5 et 8% d'extrait non alcoolique de propolis et/ou entre 1 et 8% de miel, en poids par rapport au poids total de la solution ionique

A titre d'émulsifiants, la solution ionique selon l'invention comprend des esters de saccharose et d'acide gras. Ce sont des tensioactifs non ioniques constitués d'un groupe hydrophile, le saccharose, et d'un groupe lipophile, l'acide gras. Le saccharose comportant huit fonctions hydroxyles, il est possible de produire une gamme d'esters depuis le monoester de saccharose jusqu'à l'octaester de saccharose. Ils sont non toxiques, non irritants et présentent une excellente biodégradabilité.

Les esters de saccharose et d'acide gras convenant dans le cadre de l'invention sont des tensioactifs non ioniques d'origine végétale. Ils possèdent un HLB supérieur à 10, préférentiellement entre 12 et 15 sur l'échelle de Griffin (Griffin WC, Classification of Surface-Active Agents by HLB, Journal of the Society of Cosmetic Chemists 1 (1949): 311). Ils présentent ainsi une propriété mouillante efficace et s'avèrent être des agents émulsifiants efficaces pour solubiliser dans le solvant aqueux riche en sels minéraux, tel que l'eau de mer, des composés initialement non miscibles dans ce type de solvant, tel que le miel ou l'extrait de propolis ou encore des huiles essentielles comme celles présentes dans les variantes de l'invention décrites dans ce qui suit. Il est à noter que la sélection de tensioactifs, d'une part aptes à solubiliser des composés non miscibles dans le solvant aqueux selon l'invention et, d'autre part, non irritants et ne procurant pas un mauvais goût ressenti par l'utilisateur, s'est avérée difficile. A titre de comparaison, des phospholipides cationiques d'origine naturelle ne sont pas adaptés dans le cadre de la présente invention car ils procurent un goût prononcé non acceptable une fois délivré au niveau de la cavité orale ou de la gorge.

Préférentiellement, la solution ionique selon l'invention comprend entre 0,01 et 1,00% d'un ester de saccharose et d'acide gras en C8-C24, en poids par rapport au poids total de la solution ionique.

Préférentiellement encore, l'ester de saccharose et d'acide gras est du laurate de saccharose. Le laurate de saccharose présente en effet une très bonne efficacité mouillante par rapport aux autres esters de saccharose et d'acides gras.

D'autres esters de saccharose sont également envisagés parmi lesquels le caproate de saccharose, le caprylate de saccharose, le caprate de saccharose, le myristate de saccharose, le palmitate de saccharose, le stéarate de saccharose ou le ester de saccharose et d'acide gras de coco.

La solution ionique aqueuse selon l'invention se présente ainsi sous la forme d'une émulsion stable, c'est-à-dire un mélange macroscopiquement homogène et microscopiquement hétérogène de deux substances liquides non miscibles, comme l'huile et l'eau.

Selon une autre caractéristique de l'invention, la solution ionique aqueuse présente une viscosité d'au moins 15 mPa.s, préférentiellement comprise entre 15 mPa.s et 100 mPa.s. La mesure est réalisée dans un bécher de 400 ml rempli à un minimum de 200 ml par un appareil Brookfield RVT, module 1, vitesse 10, en mesure directe, à 20°C et après 1 minute. La viscosité de la solution est supérieure à celle de l'eau pure (1 mPa.s à 20°C), par référence. Elle est équivalente à celle d'une huile végétale (environ 20 mPa.s). Ainsi, la solution ionique selon l'invention ne présente pas les caractéristiques d'un gel, c'est à dire généralement une composition gélifiée par l'ajout de polymères. A ce titre, elle est dépourvue de polymères ou gommes permettant la mise en place d'une structure sous forme de gel. Une fois appliquée au niveau de la gorge, elle ne procure pas un effet étouffant et gênant que peut procurer un gel. Par ailleurs, elle demeure apte à être délivrée en spray ou pulvérisation par un dispositif approprié, ce que ne permet pas un gel de manière optimale.

La solution ionique selon l'invention, une fois délivrée dans la gorge, tapisse et adhère aux muqueuses. Elle présente un caractère filmogène. Cette caractéristique lui confère la propriété d'agir sur une longue durée, à la fois au niveau de la douleur, mais aussi au niveau physiologique. En effet, le contact prolongé de la solution sur les muqueuses permet à l'ensemble des composants qu'elle contient d'exercer leurs actions. Par comparaison, une solution sans viscosité ou présentant une viscosité équivalente à celle de l'eau pure ne pourra agir au niveau des muqueuses que de manière transitoire. Tel sera le cas, par exemple, d'une solution d'eau de mer hypertonique sans viscosité ayant pour action principale une action de lavement de la muqueuse. La solution selon l'invention constitue donc un véhicule optimal pour les composés actifs destinés à agir de manière immédiate ou sur une durée plus longue, au niveau des muqueuses de la gorge.

Selon un des modes de réalisation préférés de l'invention, la solution ionique aqueuse comprend, de plus, au moins une huile essentielle, préférentiellement à une teneur comprise entre 0,001 et 2%, en poids par rapport au poids total de la composition.

Préférentiellement, les huiles essentielles sont choisies parmi l'huile essentielle de Gaulthérie (Wintergreen ou Gaultheria fragrantissima), de Ravintsara (Cinnamomum Camphora), de Thym à linalol (Thymus vulgaris L. linaloliferum), de niaouli (melaleuca quinquinervia), de Romarin (Rosmarinus officinalis), de marjolaine sylvestre (Thymus mastichina L. cineolifera) ou toute huile essentielle riche en composé 1,8 cinéole.

Avantageusement, la solution ionique aqueuse selon l'invention contient de l'huile essentielle de Gaultheria, seule ou en mélange avec au moins une desdites huiles essentielles.

L'huile essentielle de Gaulthérie est utilisée généralement dans le traitement des problèmes arthritiques et musculaires, tels que les crampes, tendinites, arthrose, rhumatismes. C'est une huile composée de salicylate de méthyle (99% environ) et salicylate d'éthyle (0.07 environ). Elle est utilisée ici pour son action analgésique.

L'huile essentielle de Ravintsara, de Thym à linalol, de niaouli, sont utilisées ici pour leurs actions antivirales et antimicrobiennes. L'huile essentielle de romarin est utilisée ici pour son action cicatrisante et bactéricide. L'huile essentielle de marjolaine sylvestre est utilisée ici pour son action expectorante, décongestionnante et anti-infectieuse.

De manière avantageuse, la solution ionique comprend toute huile essentielle riche en composé 1,8 cinéole. En effet, un tel composé permet d'accélérer le battement ciliaire, en plus de ses propriétés antimicrobiennes. Le battement des cils à la surface de la muqueuse est nécessaire pour éliminer le mucus chargé de pathogènes. Cette action se combine avec celle des autres composants de la solution, notamment avec l'action de la solution hypertonique. En effet, l'activité osmotique de celle-ci permet de faire sortir l'eau des cellules de la muqueuse et d'abaisser la viscosité du mucus afin de l'éliminer. En parallèle, les pathogènes sont chassés des muqueuses grâce aux huiles essentielles riches en composé 1,8 cinéole et se retrouvent en solution dans le mucus rendu fluide. Ils sont alors éliminés avec le mucus facilement.

Généralement, chaque huile essentielle est ajoutée à une teneur comprise entre 0,001 et 2 %, en poids par rapport au poids total de la solution ionique aqueuse.

Les huiles essentielles sont insolubles dans l'eau. Elles peuvent être solubilisées à l'aide d'un pourcentage élevé de solubilisants (ou tensio-actifs) mais qui ont l'inconvénient d'être irritants. Afin d'éviter ce phénomène tout en assurant une bonne dispersion des huiles essentielles, de manière stable dans le temps, le choix du composé solubilisant s'est porté, après étude, comme vu précédemment, sur un ester de saccharose et d'acide gras. En outre, la forte concentration de glycérine améliore encore la dispersion des huiles essentielles dans une solution saline, en particulier dans l'eau de mer, et contribue à les maintenir en suspension dans le produit fini. Les essais de dispersion réalisés sont reproduits dans le tableau 1 ci-dessous.

**Tableau 1**

| Essais | Stabilité |
| --- | --- |
| Glycérine + Huiles Essentielles + solution d'eau de mer hypertonique | Instable Obtention d'une solution opaque, les huiles essentielles sont mal dispersées et remontent en surface |
| Huiles Essentielles + ester de saccharose (Laurate de saccharose) + Glycérine + Eau de mer hypertonique | Stable Obtention d'une solution légèrement opalescente, les huiles essentielles restent en suspension |

A noter que la stabilité de cette dispersion est dépendante de la quantité d'eau ajoutée. Dans le cadre de l'invention, la quantité de solution saline est importante, généralement entre 20 et 40%, en pourcentage massique.

Selon un mode de réalisation de l'invention, la solution ionique aqueuse présente, de plus, un pH compris entre 4 et 6, préférentiellement entre 4,1 et 5,5, plus préférentiellement entre 4,5 et 5,5.

Le pH acide de la solution participe à son action antibactérienne. De plus, un tel pH participe, avec la forte teneur en polyols de la solution selon l'invention, à la protection de celle-ci contre les contaminations primaires et secondaires expliquées précédemment. La plupart des micro-organismes ont leur pH optimum de croissance aux environs de la neutralité (pH de 6 à 8). Un pH inférieur à 4,5-5,0 ou supérieur à 8,0-8,5 peut inhiber la multiplication de la majorité des bactéries et participer à la protection du produit. Cependant, la protection vis-à-vis des champignons est moindre dans le cas d'un pH acide. En conséquence, le pH de la solution sera tamponné préférentiellement à environ 4,5. Un test, selon la méthode pharmacopée Européenne 7ème édition, 2011 (capacité de réduction du nombre de bactéries, levures et moisissures en fonction du temps Chap. 5.1.3), a pu permettre de vérifier l'autoprotection de cette formule (exemple à suivre).

Un agent régulateur de pH peut être un acide ou un sel d'acide couramment utilisé dans l'industrie alimentaire tel que l'acide citrique, l'acide citrique monohydraté, le citrate de sodium trisodique.

Selon un mode de réalisation préféré de l'invention, la solution ionique aqueuse contient :

- 20 à 40 % d'une solution saline, de préférence d'eau de mer non diluée, d'une solution hypertonique de sels minéraux présentant une osmolalité supérieure à 350 mOsm/kg, en particulier à base d'eau de mer pure ou diluée,
- au moins 50 % de glycérol,
- entre 0,5 et 8% de sorbitol,
- entre 0,5 et 8% d'un extrait de propolis,
- entre 1 et 8% de miel,
- entre 0,01 et 1,00% d'un ester de saccharose,
- entre 0,5 et 8% d'alcool éthylique à 96 %,
- entre 0 et 2%, préférentiellement entre 0,001 et 2%, d'une ou plusieurs huiles essentielles, de Gaulthérie, de thym à linalol, de Ravintsara, de niaouli, de romarin, de marjolaine sylvestre ou toute huile essentielle riche en composé 1,8 cinéole,
- entre 0,1 et 2% d'un ou plusieurs arômes,
- entre 0,01 et 0,20% d'un agent régulateur de pH.

La solution saline utilisable est bien entendue telle que décrite précédemment. Les arômes peuvent être ceux couramment utilisés dans l'industrie alimentaire, en particulier l'arôme citron ou menthol. On soulignera l'absence d'agents conservateurs.

Préférentiellement, la solution ionique aqueuse ne contient pas d'autres composants que ceux précités.

De manière encore plus préférée, cette solution ionique aqueuse contient entre 0,001 et 0,15 % d'huile essentielle de Gaulthérie.

Ce mode de réalisation préféré de l'invention présente l'avantage de résoudre la totalité des inconvénients des produits de l'état de la technique et remplit tous les objectifs de l'invention.

Le Tableau 2 ci-dessous résume les principaux avantages des solutions ioniques aqueuses selon l'invention par comparaison avec les produits de l'état de la technique.

## Tableau 2

| Pathologie, Symptômes, manifestations | Action recherchée | produits Antiseptiques | produits Anesthésiques et antiseptiques | produits phytothérapie | Solutions ionique aqueuse selon l'invention |
|---|---|---|---|---|---|
| Mal de gorge, déglutition difficile, gorge sèche, sensation de brûlures, toussotements, se racle la gorge | Action longue durée | Non | Oui | Non | - Galénique visqueuse, huileuse, adhérente, type filmogène=> pour un contact prolongé et un effet longue durée |
| | Action immédiate Action émolliente | | | | - Formule hypertonique d'eau de mer ou de solutions salines hypertoniques (20% au moins) => effet anti-œdème<br><br>-Emulsion => action émolliente, humectante |
| Rhino-pharyngite Pharyngite Angine d'origine virale | Action antivirale Action immunostimulante | Oui | Oui | Oui | - Association d'huiles essentielles aux propriétés antivirales, antibactériennes, très bien tolérées par les muqueuses et les voies respiratoires riches en 1,8 cinéole |
| Risque de surinfection | Action antiseptique | Oui | Oui | | - pH acide<br><br>- Huiles essentielles aux propriétés antivirales, antibactériennes |
| Mal de gorge, douleurs à la déglutition (dysphagie) | Action anti douleur ou antalgique (sans être anesthésiante) Action anti-inflammatoire | Non | Oui mais risque de fausse route | | - Galénique adhérante<br>- Huile essentielle Gaultheria fragrantissima<br>- Glycérine végétale<br>- Neosorb<br>- Propolis<br>- Miel<br><br>Pas de risque de fausse route au carrefour oropharyngé (pas d'anesthésique) |
| Gorge sèche, sensation de brûlures | Action adoucissante/calmante/apaisante | Non | Non | Oui | - Miel<br>- Propolis<br>- Galénique adhérante |
| Toux, toussotements, raclement de gorge lors de la formation de mucus | Action mucolytique Anti catarrhale /fluidifiante toux grasse | Non | Non | | - Hypertonique<br>- la solution saline, notamment l'eau de mer grâce à sa richesse en minéraux lyse le mucus, favorise la clairance muco-ciliaire et participe aux processus de cicatrisation et d'immunité de la muqueuse |
| Absence de déséquilibre de la flore de cavité buccale, pas de brûlure, pas de perturbation des battements muco-ciliaires | | | | | - sans conservateurs |

L'invention concerne encore une solution ionique aqueuse telle que définie précédemment pour son utilisation dans le soin de la gorge et le traitement de la douleur de la gorge, en particulier due à une infection bactérienne ou virale, plus particulièrement due à une angine, une pharyngite, une rhinopharyngite, une bronchite, une sinusite, une rhinite, ainsi qu'une méthode de soin de la gorge et de traitement de la douleur de la gorge, en particulier due à une infection bactérienne ou virale, qui consiste à pulvériser, notamment sous forme de spray, une solution ionique aqueuse telle que définie précédemment.

L'invention est décrite et illustrée dans ce qui suit par l'intermédiaire d'exemples de réalisation.

**Exemple** 1 : **solutions ioniques aqueuses pour enfant (moins de 6 ans) ou adulte (plus de 6 ans)**

Des solutions ioniques aqueuses selon l'invention sont préparées à l'aide des ingrédients mentionnés dans le Tableau 3. Les quantités sont exprimées en pourcentage massique.

**Tableau 3**

| Ingrédients | Solution adulte | Solution enfant |
|---|---|---|
| Glycerine (glycerol) | Qsp 100 > 50 | Qsp 100 >50 |
| Propolis | 0,5-8,0 | 0,5- 8,0 |
| Neosorb 70/70B (Sorbitol) | 0,5- 8,0 | 0,5- 8,0 |
| Miel | 1,0-8,0 | 1,0-8,0 |
| Menthol | 0,001-0,01 | |
| Alcool éthylique 96% | 0,5-8,0 | |
| Arômes | 0,1-2,0 | 0,1-2,0 |
| HE dont HE Gaultheria fragrantissima | 0,001-2,000 | |
| Esters de saccharose | 0,01-1,00 | 0,05-1,00 |
| Eau de mer Hypertonique | 20,0-40,0 | 25,0-40,0 |
| Agent régulateur de pH | 0,01-0,20 | |

Préparation du solvant aqueux :

On a préparé, par électrodialyse, une solution à base d'eau de mer à teneur en sel à 22g/l, présentant une osmolalité supérieure à 350 mOsm/kg, c'est-à-dire, une solution hypertonique. Le procédé de réalisation de cette solution à base d'eau de mer, connue de l'art antérieur, est rappelé ci-dessous.

Successivement : on prélève, en tant que matière première, de l'eau de mer d'une teneur en sels supérieure à 32g/l, avantageusement par une profondeur de 5 à 10 mètres dans une zone à forts mouvements de courant, par exemple au large de Saint-Malo. On clarifie, on filtre et on analyse cette eau : si l'osmolalité de l'eau de mer clarifiée et filtrée est supérieure à l'osmolalité souhaitée, on la désode par électrodialyse jusqu'à l'obtention de l'osmolalité souhaitée, si l'osmolalité de l'eau de mer décantée est inférieure à l'osmolalité souhaitée, on la concentre jusqu'à l'obtention de l'osmolalité souhaitée, puis on ajuste les concentrations ioniques des différents ions par électrodialyse sélective, on filtre et on stocke le produit dans des conditions stériles. Les concentrations ioniques seront ajustées de manière à présenter les caractéristiques d'une « solution hypertonique de référence » telle que décrite précédemment.

Préparation des solutions ioniques:

Après la pesée de chaque matière première, les préparations des solutions ioniques sont réalisées en plusieurs phases, ces phases pouvant être différentes en fonction de l'équipement industriel utilisé.

**Préparation de la phase A :**

- Incorporer la Glycérine dans la cuve de mélange.
- Ajouter successivement sous agitation, les ingrédients suivants :

  • Propolis
  • Néosorb 70/70B
  • Miel d'acaccia

- Poursuivre l'agitation jusqu'à solubilisation complète.

**Préparation de la phase B :**

- Dans une cuve annexe, incorporer l'Alcool éthylique 96% et le Menthol puis mélanger jusqu'à dissolution du Menthol. Introduire ensuite l'ester de saccharose.
- Ajouter successivement chaque huile essentielle et arôme en prenant soin de bien mélanger entre chaque incorporation.
- Dans le cadre de la solution enfant, seuls les arômes sont ajoutés à l'ester de saccharose.

**Préparation de la phase C:**

- Incorporer l'eau de mer hypertonique (22g/l de sels) dans une 2ème cuve annexe puis ajouter l'agent régulateur de pH.

**Mélange des phases :**

- Sous agitation hélice, incorporer progressivement la phase B dans la phase A. Maintenir l'agitation.
- Ajouter progressivement la phase C.

Observations :

Les solutions se présentent sous la forme de dispersions ou d'émulsions stables. Les résultats montrent la présence de micelles (globule d'huile) d'une taille moyenne d'environ 0,6 μm (diamètre). La distribution montre que les plus petites micelles présentent un diamètre de l'ordre de 0,4 μm tandis que les plus grandes un diamètre d'environ 1,1 μm (analyse par la méthode Distribution Granulométrique par Dynamic Light Scattering inspirée de la norme ISO 22412:2008 sur un appareil Malvern Instruments - ZêtaSizer NanoZS).

**Exemple 2 : Analyse de stabilité vis à vis des contaminations bactériennes (selon méthode Pharmacopée Européenne - 7ème édition - 2011) (Tableau 4)**

**Tableau 4**

| Souche testée | Solution adulte | Solution enfant |
|---|---|---|
| Pseudomonas aeruginosa | A | A |
| Staphylococcus aureus | A | A |
| Escherichia coli | A | A |
| Candida albicans | A | A |
| Aspergillus brasiliensis | A | NC |
| **Classement** | **Conforme critères A** | **Non conforme** |
| A : résultat conforme aux critères A B : résultat conforme aux critères B NC : résultat non conforme | | |

Les souches bactériennes recherchées sont celles qui sont le plus communément rencontrées en production.
Les formules sont satisfaisantes car la contamination par Aspergillus Brasiliensis est acceptable en production. Cependant, la solution adulte 1 est plus performante à l'encontre des contaminations. En conséquence, cette formulation permet de s'affranchir d'étapes de filtration lors du procédé de fabrication, ce qui représente un gain de temps et de matériel.

La performance de la solution adulte (selon l'exemple 1) s'explique par la combinaison des polyols, des huiles essentielles en quantité supérieure, la présence d'alcool et d'un pH plus acide tamponné à 4,1-4.6. Le menthol peut également avoir un léger pouvoir antimicrobien.

Il est à noter que la formulation pour enfant, exempte d'huiles essentielles et d'alcool est efficace également à l'encontre des contaminations de par la quantité de polyols et le pH acide.

## Exemple 3 : mesure de l'activité de l'eau (Aw) (Tableau 5)

**Tableau 5**

|  | Solution adulte | Solution enfant |
|---|---|---|
| Mesure Aw | 0.664 | 0.676 |

L'activité de l'eau pure est de 1. Celle d'une solution déshydratée est de 0. Une activité de l'eau inférieure à 0,8 est satisfaisante.

Le pourcentage élevé en polyols dans la formule de la solution adulte (selon exemple 1) permet de diminuer l'activité de l'eau ce qui se traduit par une auto-protection des formules vis à vis du développement de microorganismes malgré une grande quantité de solvant aqueux.

## Exemple 4 : Test de vitesse d'écoulement

Objectif :

Mesurer la vitesse d'écoulement de la solution ionique aqueuse adulte (selon l'exemple 1) comparativement à l'eau déminéralisée et à l'eau hypertonique 22g/l afin de mettre en évidence une différence de viscosité significative permettant de démontrer l'adhésion de la solution à une surface, par exemple à la surface des muqueuses de la gorge.

Matériel utilisé :

- Ampoule à décanter de 100 ml
- Support
- Chronomètre
- Béchers
- Balance au 100$^{ème}$

Mode opératoire :

- Placer un bécher sur la balance et faire la tare
- Poser l'ampoule sur le support et la positionner au-dessus du bécher
- Amener la solution à tester à 20°C (+/- 0.5°C)
- Remplir l'ampoule à décanter avec la solution à tester (ménisque inférieur)
- Déclencher le chronomètre et ouvrir le robinet de l'ampoule à décanter simultanément
- Relever le temps écoulé ainsi que le poids délivré dans le bécher

Résultats : Tableau 6

**Tableau 6**

| Produit testé | Densité | Quantité en g | Quantité en ml | Temps écoulé (seconde) | Débit Volume (ml)/sec |
|---|---|---|---|---|---|
| Eau déminéralisée T° : 20°C | 1.00 | Test 1 - 139.04 | Test 1 - 139.04 | 21 sec 25 | 6.543 |
| | | Test 2 - 138.98 | Test 2 - 138.98 | 21 sec 03 | 6.608 |
| | | Test 3 - 138.96 | Test 3 - 138.96 | 21 sec 34 | 6.5117 |
| **Moyenne** | | **138.99** | **138.99** | **21.206** | **6.554** |
| Eau Hyper 22g/l T° 20°C | 1.008 | Test 1 - 141.45 | Test 1 - 140.33 | 22 sec 63 | 6.201 |
| | | Test 2 - 141.21 | Test 2 - 140.09 | 22 sec 46 | 6.237 |
| | | Test 3 - 141.44 | Test 3 - 140.32 | 23 sec 84 | 5.885 |
| **Moyenne** | | **141.36** | **140.25** | **22.976** | **6.1076** |
| Solution ionique aqueuse Adulte T° : 20°C | 1,15 | Test 1 - 160.55 | Test 1 - 139.60 | 36 sec 89 | 3.784 |
| | | Test 2 - 160.49 | Test 2 - 139.55 | 36 sec 06 | 3.869 |
| | | Test 3 - 160.19 | Test 3 - 139.30 | 36 sec 16 | 3.852 |
| **Moyenne** | | **160.41** | **139.48** | **36.37** | **3.835** |

**Conclusions :**

On note un écart de 0,4464 ml non significatif entre l'eau déminéralisée et l'eau de mer hypertonique 22g/l. Cet écart peut être lié à une différence de température et à la teneur en sels (hypertonique 22g/l).

L'écart de débit entre les témoins que sont l'eau déminéralisée (2,719 ml) ou l'eau hypertonique 22g/l (2,27 ml) et la Solution Adulte selon l'invention (selon l'exemple 1) est significatif, ainsi que le temps écoulé pour que l'ampoule se vide. On note également que la Solution Adulte adhère légèrement à la paroi de l'ampoule à décanter, à la manière d'une huile.

La Solution Adulte selon l'invention présente des caractéristiques de viscosité supérieures à celles de l'eau ou d'une solution d'eau de mer la rendant apte à tapisser les muqueuses et adhérer un temps utile permettant aux composés actifs d'exercer leurs actions au niveau des muqueuses.

**Exemple 5 : Test de vitesse d'écoulement n°2**

**Objectif :**

Mesurer la vitesse d'écoulement de la solution ionique aqueuse adulte (selon l'exemple 1) comparativement à une solution présente sur le marché et commercialisée sous la marque Pediakid (Laboratoires INELDEA, France) afin de mettre en évidence une différence de viscosité, d'adhésion, d'écoulement et de recouvrement significative permettant de démontrer l'adhésion et la répartition de la solution sur la muqueuse buccale.

Composition du produit Pediakid : Eau distillée d'Eucalyptus (Eucalyptus globulus), Eau distillée de Romarin (Rosmarinus officinalis), Eau de Mer à base de sel de la mer morte, Extrait d'Echinacée (echinacea purpurea), Cuivre, Extrait de Propolis, Extrait d'Aloe Vera.

**Matériel utilisé**

- Plaque support et potence
- Carton
- Chronomètre
- Pied à coulisse
- Règle
- 3 flacons de solution Adulte (selon exemple 1) conditionnés n° de lot 83361
- 3 produits Pédiakid avec embout collutoire n° de lot 12392

**Mode opératoire**

**Essai 1**

- Placer la plaque verticalement avec un angle de 105° d'inclinaison mimant le passage du carrefour oro-pharyngé.
- Tracer 2 lignes horizontalement espacées de 30 cm.
- Placer 6 points sur la ligne supérieure correspondant au point de pulvérisation initiale.
- Mesurer à l'aide de la potence et de la règle une distance de 10 cm entre la position du flacon et la zone d'impact sur la plaque.
- Simultanément, déclencher le chrono et pulvériser 2 sprays sur la zone d'impact.
- Relever le temps écoulé mis par le liquide pour atteindre la ligne inférieure.
- Répéter l'opération pour chaque produit 3 fois avec un nettoyage de la plaque après chaque série de 6.

**Essai 2**

- Placer la plaque horizontalement.
- Tracer 1 ligne verticale sur le carton pour délimiter une zone pour la solution adulte (selon l'exemple 1), une zone pour la solution Pediakid.
- Mesurer à l'aide de la potence et de la règle une distance de 10 cm entre la position du flacon (embout buccal) et la zone d'impact sur le carton.
- Pulvériser 1 spray sur la zone du carton concernée.
- Entourer la zone humidifiée par le spray et prendre le diamètre du cercle ainsi formé.
- Répéter l'opération pour chaque produit.

**Résultats**

**Tableau 7 : Essai 1**

|  | 1 Essai 1 | Essai 2 | Essai 3 | Moyenne | Ecart type | Moyenne générale |
|---|---|---|---|---|---|---|
| Solution Adulte 1 (en seconde) | 91,47 | 95,88 | 94,23 | 93,86 | 2,23 | |
| Solution Adulte 2 (en seconde) | | 118,83 | 102,65 | 105,95 | 11,59 | 93,58 |
| Solution Adulte 3 (en seconde) | 80,97 | 82,65 | 79,17 | 80,93 | 1,74 | |
| | | | | | | |
| Pediakid 1 en seconde | 54,77 | 61,83 | 58,45 | 58,35 | 3,53 | |
| Pediakid 2 en seconde | 51,72 | 78,43 | 63,12 | 64,42 | 13,40 | 60,54 |
| Pediakid 3 en seconde | 59,95 | 62,24 | 54,32 | 58,84 | 4,08 | |

La comparaison des deux moyennes peut se faire selon le test de Student avec comme hypothèse nulle Ho : le temps d'écoulement des produits Solution Adulte et Pediakid est équivalent.

L'hypothèse H1 devient alors : le temps d'écoulement de la Solution Adulte est plus long que celui de la solution Pediakid.

$t$ est donné par la formule :

$$t = \frac{(m1 - m2)}{ESy}$$

Avec $ES_v$ donné par la formule :

$$\sqrt{\frac{S1^2}{n1} + \frac{S2^2}{n2}}$$

Soit t= 6,78 avec $ES_v$=4,87

En se référant à la table du t de Student, en prenant comme entrée la valeur du t ainsi calculée et comme nombre de degrés de libertés la valeur n-1 où n est le nombre de mesures soit 17 comme degrés de liberté, la valeur de p associée est de 2,12 pour α de 0,05.

**La valeur de t n'est pas comprise dans l'intervalle (-2,12 ; 2,12), on rejette donc l'hypothèse H0, les 2 produits sont donc différents en termes de temps d'écoulement.**

**Essai 2 : Tableau 8**

|  | Essai 1 | Essai 2 | Essai 3 | Moyenne | Ecart type |
|---|---|---|---|---|---|
| Solution Adulte (en mm) | 89 | 84 | 86 | 86,33 | 2,52 |
|  |  |  |  |  |  |
| Pediakid (en mm) | 35 | 25 | 37 | 32,33 | 6,43 |

La comparaison des deux moyennes peut se faire selon le test de Student avec comme hypothèse nulle Ho : l'étalement de la solution adulte de l'exemple 1 et celui de la solution Pediakid sont équivalents.

L'hypothèse H1 devient alors : l'étalement de la solution adulte de l'exemple 1 est plus important que celui de la solution Pediakid.

On calcule la valeur de t en utilisant la formule précédente : t=13,54

En regardant la table du t de Student, en prenant comme entrée la valeur du $t$ ainsi calculée et comme nombre de degrés de libertés la valeur $n$-1 où n est le nombre de mesures soit 5 comme degrés de liberté, la valeur de p associée est de 2,57 pour α de 0,05.

**La valeur de t n'est pas comprise dans l'intervalle (-2,57 ; 2,57), on rejette donc l'hypothèse H0, les 2 produits sont donc différents en termes d'étalement.**

## Conclusion

Les deux tests réalisés ont montré des différences significatives sur l'écoulement et sur la dispersion (étalement) entre les deux produits.

Il en ressort donc qu'avec une meilleure dispersion et un temps d'écoulement plus long, la solution ionique aqueuse adulte selon l'invention pourra couvrir une grande partie de la muqueuse oropharyngée et rester en contact de celle-ci le temps nécessaire à son action.

## Revendications

1. Solution ionique aqueuse, notamment destinée au soin de la gorge, comprenant :

   - à titre de solvant aqueux, au moins 20 %, en poids par rapport au poids total de la composition, d'une solution hypertonique de sels minéraux présentant une osmolalité supérieure à 350 mOsm/kg,
   - au moins 50 % d'un ou plusieurs polyols d'origine végétale, en poids par rapport au poids total de la solution ionique,
   - au moins un ester de saccharose et d'acide gras en C8-C24, en poids par rapport au poids total de la solution ionique,
   - un extrait de propolis et/ou de miel.

2. Solution ionique aqueuse selon la revendication 1, **caractérisée en ce qu'**elle comprend entre 20 et 40%, préférentiellement entre 30 et 35%, plus préférentiellement 32%, en poids par rapport au poids total de la solution ionique, de ladite solution hypertonique de sels minéraux présentant une osmolalité supérieure à 350 mOsm/kg.

3. Solution ionique aqueuse selon la revendication 1 ou 2, **caractérisée en ce que** la solution de sels minéraux est une solution à base d'eau de mer diluée ou non diluée, ou une solution saline contenant entre 22 et 25 g/L, préférentiellement entre 22 et 23 g/L, de sels minéraux dissous de sodium, de chlore, de sulfate, de magnésium, de calcium et de potassium

4. Solution ionique aqueuse selon l'une des revendications précédentes, **caractérisée en ce qu'**elle comprend entre 50 et 65% dudit ou desdits polyols d'origine végétale, en poids par rapport au poids total de la solution ionique.

5. Solution ionique aqueuse selon l'une des revendications précédentes, **caractérisée en ce que** le ou les polyols d'origine végétale sont le glycérol pour au moins 90 %, préférentiellement au moins 95 % en poids par rapport au poids total desdits polyols, et le sorbitol pour 5 à 10 %, en poids par rapport au poids total par rapport au poids total desdits polyols.

6. Solution ionique aqueuse selon l'une des revendications précédentes, **caractérisée en ce qu'**elle comprend entre 0,5 et 8% dudit extrait de propolis et/ou entre 1 et 8% de miel, en poids par rapport au poids total de la solution ionique.

7. Solution ionique aqueuse selon l'une des revendications précédentes, **caractérisée en ce qu'**elle comprend entre 0,01 et 1,00% dudit ester de saccharose et d'acide gras en C8-C24, en poids par rapport au poids total de la solution ionique.

8. Solution ionique aqueuse selon l'une des revendications précédentes, **caractérisée en ce qu'**elle comprend de plus au moins une huile essentielle, préférentiellement à une teneur comprise entre 0,001 et 2%, en poids par rapport au poids total de la solution ionique.

9. Solution ionique aqueuse selon la revendication 6, **caractérisée en ce que** les huiles essentielles sont choisies parmi l'huile essentielle de Gaulthérie, de Ravintsara, de thyma linalol, de niaouli, de Romarin, de marjolaine sylvestre ou toute huile essentielle riche en composé 1,8 cinéole.

10. Solution ionique aqueuse selon la revendication 9, **caractérisée en ce qu'**elle contient de l'huile essentielle de Gaultheria seule ou en mélange avec au moins une desdites huiles essentielles.

11. Solution ionique aqueuse selon l'une des revendications précédentes, **caractérisée en ce qu'**elle présente, de plus, un pH compris entre 4 et 6, préférentiellement entre 4,1 et 5,5, plus préférentiellement entre 4,5 et 5,5.

12. Solution ionique aqueuse selon l'une des revendications précédentes, **caractérisée en ce qu'**elle présente une viscosité à 20° d'au moins 15 mPa.s.

13. Solution ionique aqueuse selon l'une des revendications précédentes, **caractérisée en ce qu'**elle comprend, en poids par rapport au poids total de la solution ionique :

   - 25 à 40 % d'une solution hypertonique de sels minéraux présentant une osmolalité supérieure à 350 mOsm/kg, de préférence à base d'eau de mer pure ou diluée,
   - au moins 50 % de glycérol,
   - entre 0,5 et 8,0% de sorbitol,
   - entre 0,5 et 8,0% d'un extrait hydro glycériné de propolis,
   - entre 1,0 et 8,0% de miel,
   - entre 0,01 et 1,00% d'un ester de saccharose et d'acide gras en C8-C24,
   - entre 0,5 et 8% d'alcool éthylique à 96 %,
   - entre 0 et 2%, préférentiellement entre 0,001 et 2% d'une ou plusieurs huiles essentielles de Gaulthérie, de thym zygis, de Ravintsara, de niaouli, de romarin, de marjolaine sylvestre ou toute huile essentielle riche en composé 1,8 cinéole,
   - entre 0,1 et 2% d'un ou plusieurs arômes,
   - entre 0,01 et 0,2% d'un agent régulateur de pH.

14. Solution ionique aqueuse selon la revendication précédente, **caractérisée en ce qu'**elle contient entre 0,001 et 0,08 % d'huile essentielle de Gaulthérie, en poids par rapport au poids total de la solution ionique.

15. Solution ionique aqueuse telle que définie dans l'une des revendications précédentes pour son utilisation dans le soin de la gorge et le traitement de la douleur de la gorge, en particulier due à une infection bactérienne ou virale, plus particulièrement due à une angine, une pharyngite, une rhinopharyngite, une bronchite, une sinusite, une rhinite.

**Patentansprüche**

1. Wässrige ionische Lösung, insbesondere zur Rachenpflege, umfassend:

   - als wässriges Lösungsmittel, bezogen auf das Gesamtgewicht der Zusammensetzung, mindestens 20 Gew.-% einer hypertonischen Lösung aus Mineralsalzen mit einer Osmolalität von über 350 mOsm/kg,
   - bezogen auf das Gesamtgewicht der ionischen Lösung mindestens 50 Gew.-% von einem oder mehreren Polyolen pflanzlichen Ursprungs,
   - als Gewichtsanteil bezogen auf das Gesamtgewicht der ionischen Lösung mindestens einen Ester aus Saccharose und Fettsäuren mit 8 bis 24 Kohlenstoffatomen,
   - ein Propolis- und/oder Honigextrakt.

2. Wässrige ionische Lösung nach Anspruch 1, **dadurch gekennzeichnet, dass** sie, bezogen auf das Gesamtgewicht der ionischen Lösung, zwischen 20 und 40 Gew.-%, vorzugsweise zwischen 30 und 35 Gew.-%, bevorzugter 32 Gew.-% der hypertonischen Lösung aus Mineralsalzen mit einer Osmolalität von über 350 mOsm/kg umfasst.

3. Wässrige ionische Lösung nach Anspruch 1 oder 2, **dadurch gekennzeichnet, dass** die Lösung aus Mineralsalzen eine Lösung auf Basis von verdünntem oder unverdünntem Meerwasser ist oder eine Salzlösung, die zwischen 22 und 25 g/l, vorzugsweise zwischen 22 und 23 g/l gelöste Natrium-, Chlor-, Sulfat-, Magnesium-, Calcium- und Kalium-Mineralsalze enthält.

4. Wässrige ionische Lösung nach einem der vorhergehenden Ansprüche, **dadurch gekennzeichnet, dass** sie bezogen auf das Gesamtgewicht der ionischen Lösung zwischen 50 und 65 Gew.-% des oder der Polyole pflanzlichen Ursprungs umfasst.

5. Wässrige ionische Lösung nach einem der vorhergehenden Ansprüche, **dadurch gekennzeichnet, dass** das oder die Polyole pflanzlichen Ursprungs zu mindestens 90 Gew.-%, vorzugsweise mindestens 95 Gew.-%, bezogen auf das Gesamtgewicht der Polyole Glycerin und zu 5 bis 10 Gew.-% bezogen auf das Gesamtgewicht bezogen auf das Gesamtgewicht der Polyole Sorbitol sind.

6. Wässrige ionische Lösung nach einem der vorhergehenden Ansprüche, **dadurch gekennzeichnet, dass** sie bezogen auf das Gesamtgewicht der ionischen Lösung zwischen 0,5 und 8 Gew.-% des Propolisextrakts und/oder zwischen 1 und 8 Gew.-% Honig umfasst.

7. Wässrige ionische Lösung nach einem der vorhergehenden Ansprüche, **dadurch gekennzeichnet, dass** sie bezogen auf das Gesamtgewicht der ionischen Lösung zwischen 0,01 und 1,00 Gew.-% des Esters aus Saccharose und Fettsäuren mit 8 bis 24 Kohlenstoffatomen umfasst.

8. Wässrige ionische Lösung nach einem der vorhergehenden Ansprüche, **dadurch gekennzeichnet, dass** sie ferner mindestens ein ätherisches Öl umfasst, vorzugsweise mit einem Gehalt zwischen 0,001 und 2 Gew.-% bezogen auf das Gesamtgewicht der ionischen Lösung.

9. Wässrige ionische Lösung nach Anspruch 6, **dadurch gekennzeichnet, dass** die ätherischen Öle ausgewählt sind aus dem ätherischen Öl von Gaultheria, Ravintsara, Thymian Linalool, Myrtenheide, Rosmarin, Thymus mastichina oder jedem beliebigen ätherischen Öl, das reich an der Verbindung 1,8-Cineol ist.

10. Wässrige ionische Lösung nach Anspruch 9, **dadurch gekennzeichnet, dass** sie das ätherische Öl von Gaultheria allein oder mit mindestens einem der ätherischen Öle gemischt enthält.

11. Wässrige ionische Lösung nach einem der vorhergehenden Ansprüche, **dadurch gekennzeichnet, dass** sie ferner einen pH-Wert zwischen 4 und 6, vorzugsweise zwischen 4,1 und 5,5, bevorzugter zwischen 4,5 und 5,5 aufweist.

**12.** Wässrige ionische Lösung nach einem der vorhergehenden Ansprüche, **dadurch gekennzeichnet, dass** sie eine Viskosität bei 20° von mindestens 15 mPa·s aufweist.

**13.** Wässrige ionische Lösung nach einem der vorhergehenden Ansprüche, **dadurch gekennzeichnet, dass** sie in Gewichtsprozent bezogen auf das Gesamtgewicht der ionischen Lösung Folgendes umfasst:

- 25 bis 40 % einer hypertonischen Lösung aus Mineralsalzen mit einer Osmolalität von über 350 mOsm/kg, vorzugsweise auf Basis von reinem oder verdünntem Meerwasser,
- mindestens 50 % Glycerin,
- zwischen 0,5 und 8,0 % Sorbitol,
- zwischen 0,5 und 8,0 % eines Propolis-Extrakts in Wasser und Glycerin,
- zwischen 1,0 und 8,0 % Honig,
- zwischen 0,01 und 1,00 % eines Esters aus Saccharose und Fettsäuren mit 8 bis 24 Kohlenstoffatomen,
- zwischen 0,5 und 8 % 96%-igen Ethanoln,
- zwischen 0 und 2%, vorzugsweise zwischen 0,001 und 2% von einem oder mehreren ätherischen Ölen von Gaultheria, Joch-Thymian, Ravintsara, Myrtenheide, Rosmarin, Thymus mastichina oder jedem beliebigen ätherischen Öl, das reich an der Verbindung 1,8-Cineol ist,
- zwischen 0,1 und 2% von einem oder mehreren Aromen,
- zwischen 0,01 und 0,2% von einem Mittel zur pH-Regelung.

**14.** Wässrige ionische Lösung nach dem vorhergehenden Anspruch, **dadurch gekennzeichnet, dass** sie bezogen auf das Gesamtgewicht der ionischen Lösung zwischen 0,001 und 0,08 Gew.-% des ätherischen Öls von Gaultheria enthält.

**15.** Wässrige ionische Lösung nach einem der vorhergehenden Ansprüche zur Verwendung bei der Rachenpflege und der Behandlung von Rachenschmerzen, vor allem aufgrund einer bakteriellen oder viralen Entzündung, insbesondere aufgrund einer Angina, einer Rachenentzündung, einer Rhinopharyngitis, einer Bronchitis, einer Nasennebenhöhlenentzündung, einer Rhinitis.

**Claims**

**1.** Aqueous ionic solution, in particular intended for throat care, comprising:

- as aqueous solvent, at least 20%, by weight relative to the total weight of the composition, of a hypertonic solution of mineral salts having an osmolality of greater than 350 mOsm/kg,
- at least 50% of one or more polyols of plant origin, by weight relative to the total weight of the ionic solution,
- at least one sucrose ester of a $C_8$-$C_{24}$ fatty acid, by weight relative to the total weight of the ionic solution,
- an extract of propolis and/or of honey.

**2.** Aqueous ionic solution according to Claim 1, **characterized in that** it comprises between 20% and 40%, preferentially between 30% and 35%, more preferentially 32%, by weight relative to the total weight of the ionic solution, of said hypertonic solution of mineral salts having an osmolality of greater than 350 mOsm/kg.

**3.** Aqueous ionic solution according to Claim 1 or 2, **characterized in that** the solution of mineral salts is a solution based on diluted or non-diluted seawater, or a saline solution containing between 22 and 25 g/l, preferentially between 22 and 23 g/l, of dissolved sodium, chlorine, sulfate, magnesium, calcium and potassium mineral salts.

**4.** Aqueous ionic solution according to one of the preceding claims, **characterized in that** it comprises between 50% and 65% of said polyol(s) of plant origin, by weight relative to the total weight of the ionic solution.

**5.** Aqueous ionic solution according to one of the preceding claims, **characterized in that** at least 90%, preferentially at least 95% by weight, of the polyol(s) of plant origin, relative to the total weight of said polyols, is glycerol, and 5% to 10% by weight of the polyol(s) of plant origin, relative to the total weight relative to the total weight of said polyols, is sorbitol.

**6.** Aqueous ionic solution according to one of the preceding claims, **characterized in that** it comprises between 0.5% and 8% of said extract of propolis and/or between 1% and 8% of honey, by weight relative to the total weight of the ionic solution.

7. Aqueous ionic solution according to one of the preceding claims, **characterized in that** it comprises between 0.01% and 1.00% of said sucrose ester of a $C_8$-$C_{24}$ fatty acid, by weight relative to the total weight of the ionic solution.

8. Aqueous ionic solution according to one of the preceding claims, **characterized in that** it comprises in addition at least one essential oil, preferentially in a content of between 0.001% and 2% by weight relative to the total weight of the ionic solution.

9. Aqueous ionic solution according to Claim 6, **characterized in that** the essential oils are chosen from Gaultheria essential oil, Ravintsara essential oil, thyme linalool essential oil, niaouli essential oil, rosemary essential oil, Spanish marjoram essential oil or any essential oil rich in 1,8-cineole compound.

10. Aqueous ionic solution according to Claim 9, **characterized in that** it contains Gaultheria essential oil alone or as a mixture with at least one of said essential oils.

11. Aqueous ionic solution according to one of the preceding claims, **characterized in that** it has, in addition, a pH of between 4 and 6, preferentially between 4.1 and 5.5, more preferentially between 4.5 and 5.5.

12. Aqueous ionic solution according to one of the preceding claims, **characterized in that** it has a viscosity at 20° of at least 15 mPa.s.

13. Aqueous ionic solution according to one of the preceding claims, **characterized in that** it comprises, by weight relative to the total weight of the ionic solution:

- 25% to 40% of a hypertonic solution of mineral salts having an osmolality of greater than 350 mOsm/kg, preferably based on pure or diluted seawater,
- at least 50% of glycerol,
- between 0.5% and 8.0% of sorbitol,
- between 0.5% and 8.0% of a water-glycerol extract of propolis,
- between 1.0% and 8.0% of honey,
- between 0.01% and 1.00% of a sucrose ester of a $C_8$-$C_{24}$ fatty acid,
- between 0.5% and 8% of 96% ethyl alcohol,
- between 0 and 2%, preferentially between 0.001% and 2% of one or more Gaultheria, Thymus zygis, Ravintsara, niaouli, rosemary or Spanish marjoram essential oils or any essential oil rich in 1,8-cineole compound,
- between 0.1% and 2% of one or more flavourings,
- between 0.01% and 0.2% of a pH regulator.

14. Aqueous ionic solution according to the preceding claim, **characterized in that** it contains between 0.001% and 0.08% of Gaultheria essential oil, by weight relative to the total weight of the ionic solution.

15. Aqueous ionic solution as defined in one of the preceding claims, for use thereof in throat care and the treatment of throat pain, in particular due to a bacterial or viral infection, more particularly due to a sore throat, pharyngitis, rhinopharyngitis, bronchitis, sinusitis or rhinitis.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2068896 A1

- FR 2971713 A1

**Littérature non-brevet citée dans la description**

- Handbook of Chemistry and Physics. CRC PRESS, 1982, F 163
- **GRIFFIN WC.** Classification of Surface-Active Agents by HLB. *Journal of the Society of Cosmetic Chemists,* 1949, vol. 1, 311

- capacité de réduction du nombre de bactéries, levures et moisissures en fonction du temps. pharmacopée Européenne. 2011